(19) Europäisches Patentamt
European Patent Office
Office européen des brevets

(11) **EP 2 269 498 A1**

(12) **EUROPEAN PATENT APPLICATION**
published in accordance with Art. 153(4) EPC

(43) Date of publication:
**05.01.2011 Bulletin 2011/01**

(51) Int Cl.:
**A61B 1/00** *(2006.01)*    **A61B 5/07** *(2006.01)*

(21) Application number: **09733810.7**

(86) International application number:
**PCT/JP2009/055652**

(22) Date of filing: **23.03.2009**

(87) International publication number:
**WO 2009/130962 (29.10.2009 Gazette 2009/44)**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **22.04.2008 JP 2008111609**

(71) Applicant: **Olympus Corporation**
**Shibuya-ku**
**Tokyo 151-0072 (JP)**

(72) Inventor: **YOSHIZAWA, Fukashi**
**Tokyo 151-0072 (JP)**

(74) Representative: **Winter, Brandl, Fürniss, Hübner, Röss, Kaiser, Polte - Partnerschaft**
**Patent- und Rechtsanwaltskanzlei**
**Alois-Steinecker-Straße 22**
**D-85354 Freising (DE)**

(54) **LIVING BODY OBSERVING SYSTEM AND METHOD OF DRIVING LIVING BODY OBSERVING SYSTEM**

(57)    A living body observation system of the invention includes: a living body information acquiring apparatus including a living body information acquiring section for acquiring living body information in a living body, a wireless transmission section for wirelessly transmitting the living body information to outside of the living body, a power source section for supplying driving power for driving the living body information acquiring section and the wireless transmission section, a magnetic field detecting section for detecting a magnetic field from outside and outputting a detection result as an electric signal, and a power supply control section for controlling a supplying state of the driving power supplied from the power source section to the living body information acquiring section and the wireless transmission section based on the electric signal; and a magnetic field generating section disposed outside the living body information acquiring apparatus, the magnetic field generating section including a resonant circuit for generating a magnetic field by resonance, and a driving circuit for supplying driving voltage for driving the resonant circuit.

**FIG.1**

EP 2 269 498 A1

**Description**

Technical Field

The present invention relates to a living body observation system and a method of driving the living body observation system, and more particularly to a living body observation system including a power source section composed of a battery or the like and a method of driving the living body observation system.

Background Art

Conventionally, endoscopes have been widely used in the medical field or the like. Endoscopes in the medical field, in particular, are used for the purpose of observing inside of a living body. One type of the endoscopes described above is a capsule endoscope developed in recent years, which is swallowed by a test subject to be disposed in a body cavity, and capable of picking up images of a photographic subject while moving in the body cavity with peristaltic movement and wirelessly transmitting the picked-up images of the photographic subject to outside as image pickup signals.

For example, Japanese Patent Application Laid-Open Publication No. 2001-224553 discloses an apparatus having substantially the same functions as those of the capsule endoscope described above.

The Japanese Patent Application Laid-Open Publication No. 2001-224553 describes a capsule endoscope having a configuration in which a reed switch with contacts that open when placed in a magnetic field is used as a non-contact power source switch. The capsule endoscope described in the Japanese Patent Application Laid-Open Publication No. 2001-224553 is configured such that, due to the working of the above-described reed switch, the contacts of the reed switch open to turn off the power source of the endoscope when the endoscope is stored in a container or a storage case having a magnet, and the contacts close to turn on the power source (power is supplied from a battery) of the endoscope when the endoscope is taken out of the container or the storage case, for example.

However, the capsule endoscope disclosed in the Japanese Patent Application Laid-Open Publication No. 2001-224553 is configured such that the power source is turned on upon being taken out from the container or storage case having a magnet. Accordingly, consumption of the internal battery starts at a stage before the capsule endoscope is placed in a living body. As a result, the capsule endoscope disclosed in the Japanese Patent Application Laid-Open Publication No. 2001-224553 has such a problem that the remaining level of the internal battery falls to the extent that the image pickup of a desired region is impossible, before the capsule endoscope reaches the desired region in a living body, and observation of the desired region becomes impossible in some cases. In such cases, observation using the capsule endoscope is interrupted, which results in the need for a follow-up observation.

In order to once again turn off the power source of the capsule endoscope disclosed in the Japanese Patent Application Laid-Open Publication No. 2001-224553 after the power source was turned on once, there is a need for applying a magnetic field having a predetermined intensity or more, orientation of which agrees with the orientation of the reed switch, to the capsule endoscope by using a permanent magnet and the like. That is, the capsule endoscope disclosed in the Japanese Patent Application Laid-Open Publication No. 2001-224553 has a problem that cumbersome operation is required for turning off the power source of the endoscope once again, after the power source was turned on once.

The present invention has been achieved in view of the above-described circumstances, and an object of the present invention is to provide a living body observation system and a method of driving the living body observation system, in which consumption of internal battery can be controlled more easily than conventional systems and methods, due to the ease of switching between turn-on and turn-off of the power source.

Disclosure of the Invention

Means for Solving the Problem

The present invention provides a living body observation system which includes a living body information acquiring apparatus including: a living body information acquiring section for acquiring living body information in a living body; a wireless transmission section for wirelessly transmitting the living body information to outside of the living body; a power source section for supplying driving power for driving the living body information acquiring section and the wireless transmission section; a magnetic field detecting section for detecting a magnetic field from outside and outputting a detection result as an electric signal; and a power supply control section for controlling a supplying state of the driving power supplied from the power source section to the living body information acquiring section and the wireless transmission section based on the electric signal, and which also includes a magnetic field generating section disposed outside the living body information acquiring apparatus, the magnetic field generating section including a resonant circuit for generating a magnetic field by resonance and a driving circuit for supplying driving voltage for driving the resonant circuit.

The present invention also provides a method of driving the living body observation system which includes a living body information acquiring apparatus including: a living body information acquiring section for acquiring living body information in a living body; a wireless transmission section for wirelessly transmitting the living body information to outside of the living body; a power source section for supplying driving power for driving the living body information acquiring section and the wireless transmission section; a magnetic field detecting section for detecting a magnetic field from outside and outputting a detection result as an electric signal; and a power supply control section for controlling a supplying state of the driving power supplied from the power source section to the living body information acquiring section and the wireless transmission section based on the electric signal, and which also includes a magnetic field generating section disposed outside the living body information acquiring apparatus, the magnetic field generating section including a resonant circuit for generating a magnetic field by resonance and a driving circuit for supplying driving voltage for driving the resonant circuit. The method includes switching a power source state of the living body information acquiring apparatus between on and off, every time the magnetic field is generated from the magnetic field generating section.

Brief Description of the Drawings

FIG. 1 is a view showing a configuration of a main part of a living body observation system according to a first embodiment of the present invention.

FIG. 2 is a view showing an example of a specific configuration of a magnetic field generating section according to the first embodiment of the present invention.

FIG. 3 is a view showing an example of a specific configuration of a power supplying section and a magnetic field detecting section according to the first embodiment of the present invention.

FIG. 4 is a timing chart showing an operating state of the magnetic field generating section according to the first embodiment of the present invention.

FIG. 5 is a timing chart showing a correlation between an operating state of the power supplying section and a power source state of a capsule endoscope according to the first embodiment of the present invention.

FIG. 6 is a timing chart showing an operating state of a magnetic field generating section according to a second embodiment of the present invention.

FIG. 7 is a timing chart showing a correlation between an operating state of a power supplying section and a power source state of a capsule endoscope according to the second embodiment of the present invention.

Best Mode for Carrying Out the Invention

Hereinafter, embodiments of the present invention will be described with reference to the drawings.

(First Embodiment)

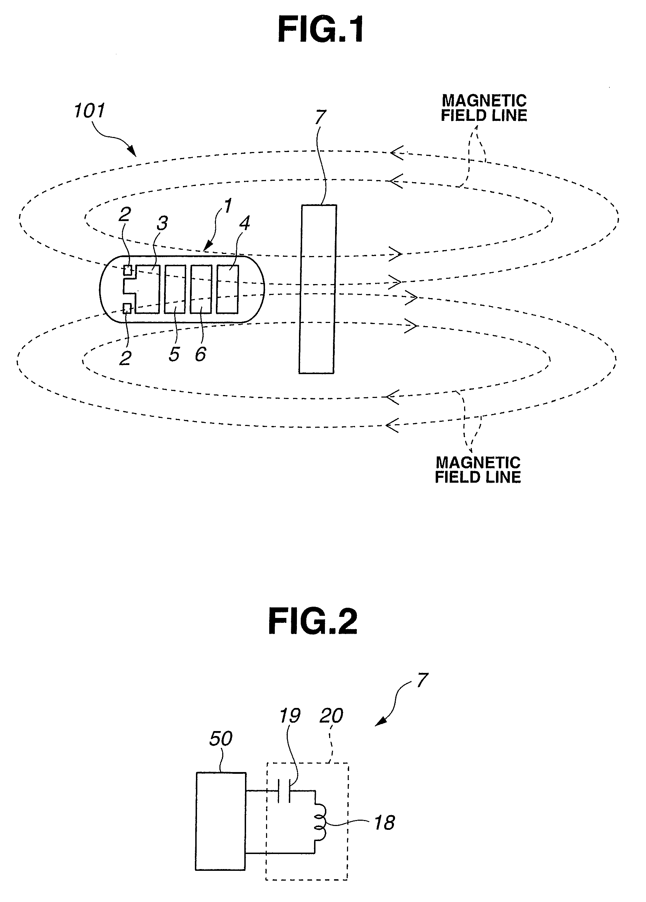

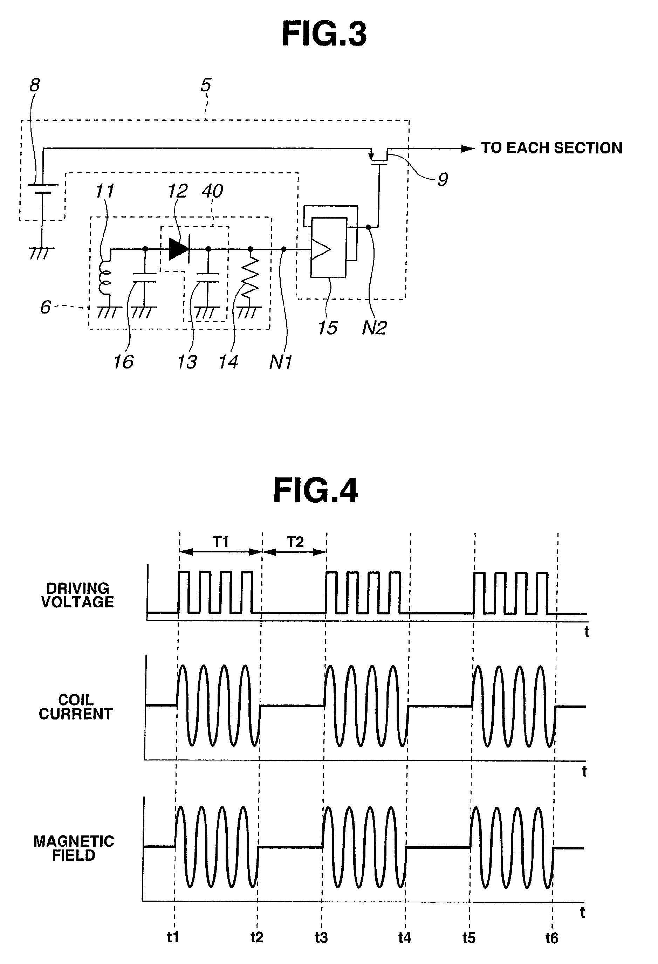

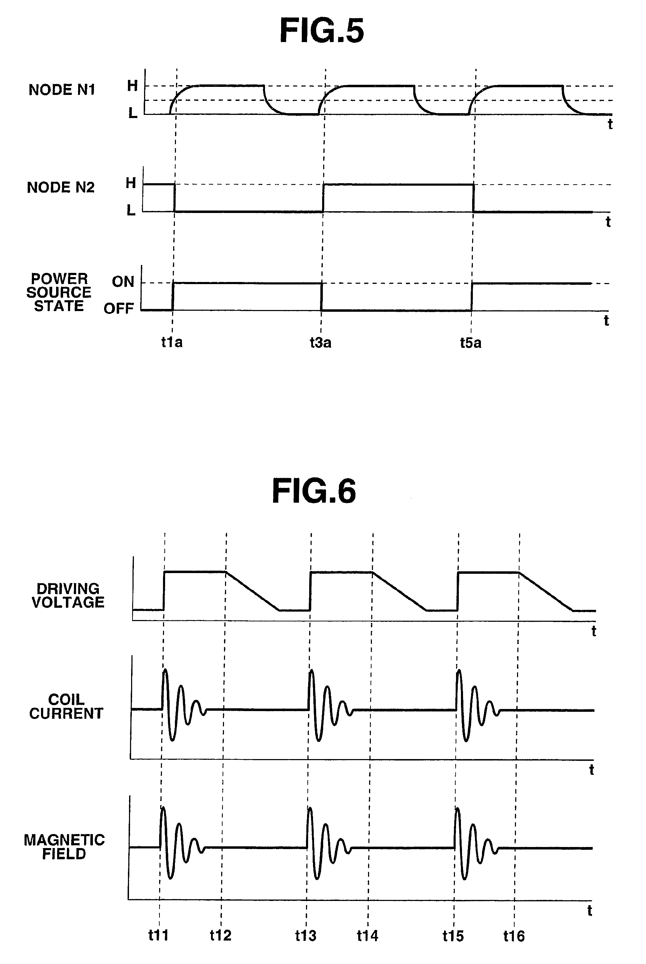

FIGS. 1 to 5 relate to the first embodiment of the present invention. FIG. 1 is a view showing a configuration of a main part of a living body observation system according to the first embodiment of the present invention. FIG. 2 is a view showing an example of a specific configuration of a magnetic field generating section according to the first embodiment of the present invention. FIG. 3 is a view showing an example of a specific configuration of a power supplying section and a magnetic field detecting section according to the first embodiment of the present invention. FIG. 4 is a timing chart showing an operating state of the magnetic field generating section according to the first embodiment of the present invention. FIG. 5 is a timing chart showing a correlation between an operating state of the power supplying section and a power source state of a capsule endoscope according to the first embodiment of the present invention.

As shown in FIG. 1, a living body observation system 101 includes a capsule endoscope 1 having a size and a shape which can be disposed in a living body, and a magnetic field generating section 7 that generates a magnetic field outside the capsule endoscope 1.

As shown in FIG. 1, the capsule endoscope 1 includes inside thereof: an illuminating section 2 for emitting illumination light for illuminating a photographic subject in a living body; an image pickup section 3 for picking up an image of the photographic subject illuminated by the illuminating section 2 and outputting the picked-up image as an image pickup signal; a wireless transmission section 4 for wirelessly transmitting the image pickup signal outputted from the image pickup section 3 to outside of the living body; a power supplying section 5 for supplying driving power required for driving each of the illuminating section 2, the image pickup section 3 and the wireless transmission section 4; and a magnetic field detecting section 6 capable of detecting the magnetic field generated by the magnetic field generating section 7.

That is, a living body information acquiring section of the present embodiment is configured by including the illuminating section 2 and the image pickup section 3.

The magnetic field generating section 7 has a configuration in which generation state of the magnetic field can be switched between on and off in response to a user's operation of a switch or the like, not shown.

Furthermore, as shown in FIG. 2, the magnetic field generating section 7 is configured by including a resonant circuit 20 and a driving circuit 50 for driving the resonant circuit 20. The resonant circuit 20 includes a magnetic field generating coil 18 and a resonant capacitor 19.

Note that, when inductance of the magnetic field generating coil 18 is L1 and capacitance of the resonant capacitor 19 is C1, the value of a resonant frequency fr1 of the resonant circuit 20 is calculated based on the following mathematical expression (1). In addition, in the magnetic field generating section 7 of the present embodiment, the values of the inductance L1 and the capacitance C1 are set such that the resonant frequency fr1 and the frequency f1 of the driving voltage supplied from the driving circuit 50 agree with each other.

$$fr1 = 1 \Big/ 2\pi\sqrt{L1 \cdot C1} \quad \cdot\ \cdot\ \cdot \quad (1)$$

On the other hand, the power supplying section 5 is configured by including a power source section 8 composed of a battery or the like, a P-channel FET 9, and a frequency divider circuit 15 for dividing an output signal from the magnetic field detecting section 6 by two, as shown in FIG. 3.

The P-channel FET 9 has the source connected to the power source section 8, the gate connected to an output end of the frequency divider circuit 15, and the drain connected to each of the illuminating section 2, the image pickup section 3, and the wireless transmission section 4.

Note that, the power supplying section 5 is not limited to one configured by using the P-channel FET 9 and may be one configured by using an electronic switch or the like having the similar switching function as that of the P-channel FET 9.

The magnetic field detecting section 6 is configured by including a magnetic field detecting coil 11 for outputting an electric signal corresponding to the magnetic field generated by the magnetic field generating section 7, a rectifying section 40 for rectifying the electric signal outputted from the magnetic field detecting coil 11 and outputting the rectified electric signal, a resistor 14, and a resonant capacitor 16.

Note that the magnetic field detecting coil 11 may be formed of, for example, a solenoid coil or a planar coil, and may have any shape as long as the magnetic field detecting coil 11 can be disposed in the capsule endoscope 1.

The rectifying section 40 has a diode 12, an input end of which is connected to an output end of the magnetic field detecting coil 11, and a smoothing capacitor 13 for smoothing an electric signal outputted from the diode 12. Note that the rectifying section 40 in the present embodiment is not limited to one for performing half-wave rectification, and may be one for performing full-wave rectification.

The resistor 14 is connected to an output end of the diode 12 in parallel with the smoothing capacitor 13.

The resonant capacitor 16 is connected to the input end of the diode 12 in parallel with the magnetic field detecting coil 11.

Note that when inductance of the magnetic field detecting coil 11 is L2 and capacitance of the resonant capacitor 16 is C2, the value of a resonant frequency fr2 of a resonant circuit composed of the magnetic field detecting coil 11 and the resonant capacitor 16 is calculated based on the following mathematical expression (2). In addition, in the magnetic field detecting section 6 of the present embodiment, the values of the inductance L2 and the capacitance C2 are set such that the resonant frequency fr2 and the frequency f1 of the driving voltage supplied from the driving circuit 50 agree with each other.

$$fr2 = 1 \Big/ 2\pi\sqrt{L2 \cdot C2} \quad \cdot\ \cdot\ \cdot \quad (2)$$

Now description will be made on operations of the power supplying section 5, the magnetic field detecting section 6, and the magnetic field generating section 7 according to the present embodiment.

First, as shown in FIG. 4, the driving circuit 50 continuously generates driving voltage of a rectangular waveform at the frequency f1 during the period from time t1 to time t2 (period T1). The driving voltage at the frequency f1 is then supplied to the magnetic field generating coil 18 and the resonant capacitor 19.

In the magnetic field generating section 7 of the present embodiment, the values of inductance L1 and the capacitance C1 are set such that the resonant frequency fr1 and the frequency f1 agree with each other. According to this setting, in the magnetic field generating section 7 of the present embodiment, when setting the magnitude of the driving voltage of the driving circuit 50, loss only due to a resistance component of parasitic elements generated in the resonant circuit 20 has only to be considered. As a result, the magnetic field generating section 7 of the present embodiment is capable of generating a magnetic field while suppressing the power consumption.

In addition, as shown in FIG. 4, the driving circuit 50 stops generating the driving voltage during the period from time t2 to time t3 (period T2).

The driving circuit 50 repeatedly generates and stops the driving voltage as described above also after the time t3.

On the other hand, as shown in FIG. 4, during the period from the time t1 to the time t2, the driving voltage generated in the driving circuit 50 is supplied to the resonant circuit 20, and thereby resonance at the resonant frequency fr1 is generated in the resonant circuit 20 and alternating coil current flows through the magnetic field generating coil 18.

As a result, the magnetic field generating coil 18 generates a magnetic field at the frequency f1 which is the magnetic field corresponding to the coil current during the period from the time t1 to the time t2. Note that the magnetic field generating coil 18 does not generate the above-described magnetic field in the period from the time t2 to the time t3, during which the supply of the driving voltage from the driving circuit 50 is stopped.

The magnetic field generating coil 18 in the resonant circuit 20 repeatedly generates and stops the magnetic field at the frequency f1 as described above also after the time t3.

On the other hand, when the magnetic field generating section 7 starts generating the magnetic field at the time t1, an electric potential difference is generated between both ends of the magnetic field detecting coil 11 by electromagnetic induction, and thereafter an alternating-current electric signal corresponding to the electric potential difference is outputted to the rectifying section 40.

The alternating-current electric signal outputted from the magnetic field detecting coil 11 is then rectified in the rectifying section 40 and thereby converted into a direct-current electric signal and outputted to an input end of the frequency divider circuit 15. In response to this, the electric potential level of a node N1 is shifted from a low (hereinafter referred to as L) level to a high (hereinafter referred to as H) level at a time t1 a which is a timing immediately after the time t1, as shown in FIG. 5.

After that, when the magnetic field generating section 7 stops generating the magnetic field at the time t2, an electric charge accumulated in the smoothing capacitor 13 is discharged via the resistor 14. With the electric discharge, the electric potential level of the node N1 is shifted from the H level to the L level as shown in FIG. 5.

That is, at the node N1 on the output end side of the magnetic field detecting section 6 of the present embodiment, the electric potential level is shifted from the L level to the H level in the period T1 during which the magnetic field is generated from the magnetic field generating section 7. The electric potential level is shifted from the H level to the L level in the period T2 during which the magnetic field is not generated from the magnetic field generating section 7.

Furthermore, an output signal having the electric potential level of the node N1 is inputted to the input end of the frequency divider circuit 15. In response to the signal, the electric potential level of a node N2 on the output end side of the frequency divider circuit 15 is shifted from the H level to the L level at the time t1a.

Following the shift of the electric potential level of the node N2 from the H level to the L level, the P-channel FET 9 is shifted from an off-state to an on-state, and thereby the power source section 8 starts supplying driving power to each of the illuminating section 2, the image pickup section 3, and the wireless transmission section 4. That is, as shown in FIG. 5, the power source of the capsule endoscope 1 is turned on at the time t1a.

As shown in FIG. 5, the electric potential level of the node N2 is maintained at the L level, even when the electric potential level of the node N1 was shifted from the H level to the L level. Accordingly, the electric potential level of the node N2 remains at the L level until the time t3a which is the timing when the electric potential level of the node N1 is shifted again from the L level to the H level and the timing almost immediately after the time t3. As a result, the capsule endoscope 1 maintains the on-state during the period from the time t1a to the time t3a, as shown in FIG. 5.

In addition, as shown in FIG. 5, the electric potential level of the node N2 is shifted from the L level to the H level at the time t3a. Following the level shift, the P-channel FET 9 is shifted from the on-state to the off state, and thereby the power source section 8 stops supplying the driving power to each of the illuminating section 2, the image pickup section 3, and the wireless transmission section 4. As a result, the power source of the capsule endoscope 1 is turned off at the time t3a, as shown in FIG. 5.

That is, the capsule endoscope 1 of the present embodiment has a configuration and working such that on/off switching of the power source is performed every time the generation state of the magnetic field is switched from off to on in the magnetic field generating section 7.

Accordingly, the living body observation system 101 of the present embodiment enables easy on/off switching of the power source of the capsule endoscope 1 at a user's desired timing, thereby enabling easier control of exhaustion of the power source section 8 of the capsule endoscope 1 compared with a conventional system.

In addition, the living body observation system 101 of the present embodiment is configured such that the frequency f1 of the magnetic field generated from the magnetic field generating section 7 agrees with the resonant frequency fr2 of the resonant circuit composed of the magnetic field detecting coil 11 and the resonant capacitor 16. Accordingly, the living body observation system 101 of the present embodiment enables the magnetic field detecting section 6 to have improved detection sensitivity to the magnetic field generated from the magnetic field generating section 7 and reduced detection sensitivity to an unintended disturbance magnetic field. As a result, the living body observation system 101 of the present embodiment can stably and surely perform the on/off switching of the power source of the capsule endoscope 1.

Furthermore, the living body observation system 101 of the present embodiment can reduce the driving voltage required when the magnetic field is generated by the magnetic field generating section 7.

Note that the on/off switching of the power source of the capsule endoscope 1 can be similarly performed not only in the case where the capsule endoscope 1 is disposed in the living body but also in the case where the capsule endoscope 1 is disposed outside the living body.

(Second Embodiment)

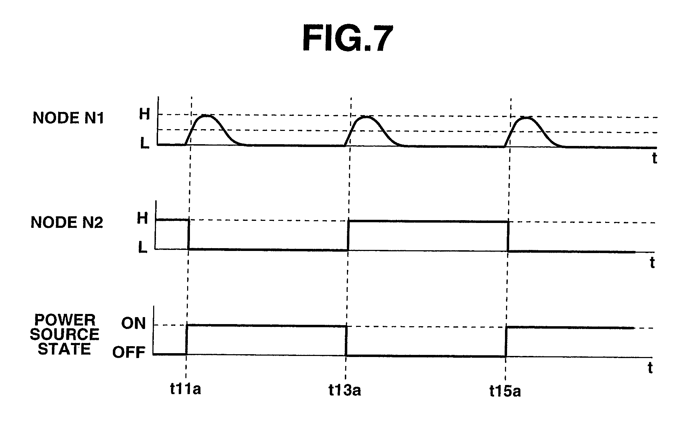

FIGS. 6 and 7 relate to the second embodiment of the present invention. FIG. 6 is a timing chart showing an operating state of a magnetic field generating section according to the second embodiment of the present invention. FIG. 7 is a timing chart showing a correlation between an operating state of the power supplying section and the power source state of the capsule endoscope according to the second embodiment of the present invention.

Note that a living body observation system of the present embodiment has almost the same configuration as that of the living body observation system 101 of the first embodiment. Accordingly, in the present embodiment, description will be mainly made on the sections that perform operations different from those performed by the sections in the living body observation system 101 of the first embodiment.

Now description will be made on operations performed by a power supplying section 5, a magnetic field detecting section 6, and a magnetic field generating section 7 of the present embodiment.

First, a driving circuit 50 generates step-like driving voltage at a time t11 as shown in FIG. 6. The step-like driving voltage is then supplied to the magnetic field generating coil 18 and the resonant capacitor 19.

The driving voltage generated by the driving circuit 50 is supplied to the resonant circuit 20, and thereby resonance at the resonant frequency fr1 is generated in the resonant circuit 20 and alternating coil current starts flowing through the magnetic field generating coil 18.

The alternating coil current flowing through the magnetic field generating coil 18 is gradually attenuated by the loss due to the resistance component parasitic on the resonant circuit 20 and becomes zero no later than the time t12, as shown in FIG. 6. That is, the coil current flows through the magnetic field generating coil 18 only during a predetermined period after the timing when the driving voltage was supplied from the driving circuit 50, the predetermined period being shorter than the period from the time t11 to the time t12.

Moreover, the magnetic field generating coil 18 generates the magnetic field corresponding to the alternating coil current flowing therethrough, as shown in FIG. 6. That is, the magnetic field generated by the magnetic field generating coil 18 is gradually attenuated with the attenuation of the coil current flowing through the magnetic field generating coil 18 itself and becomes zero no later than the time t12.

On the other hand, at the time t12 after the coil current flowing through the magnetic field generating coil 18 became zero, the driving circuit 50 gradually decreases the driving voltage supplied to the resonant circuit 20 at a frequency sufficiently lower than the resonant frequency fr1 of the resonant circuit 20, as shown in FIG. 6. As a result, the magnetic field generating coil 18 is prevented from generating the magnetic field during the period from the time t12 until the driving voltage is fully decreased.

By performing the above-described operations, the magnetic field generating section 7 of the present embodiment can limit the period during which power is consumed to the period during which the step-like driving voltage is generated from the driving circuit 50. As a result, the present embodiment can reduce the power consumption in the magnetic field generating section 7.

In addition, as described above, the driving circuit 50 of the present embodiment has a relative simple configuration for generating step-like driving voltage. Therefore, the present embodiment enables the relative simple configuration of the magnetic field generating section 7.

When the magnetic field generating section 7 starts generating a magnetic field at the time t11, an electric potential difference is generated between both ends of the magnetic field detecting coil 11 by electromagnetic induction, and thereafter an alternating-current electric signal corresponding to the electric potential difference is outputted to the rectifying section 40.

The alternating-current electric signal outputted from the magnetic field detecting coil 11 is then rectified in the rectifying section 40 and thereby converted into a direct-current electric signal and outputted to an input end of the frequency divider circuit 15. In response to this, the electric potential level of the node N1 is shifted from the L level to the H level at the time t11 a which is the timing almost immediately after the time t11, as shown in FIG. 7.

After that, when the magnetic field generated from the magnetic field generating section 7 is further attenuated, an electric charge accumulated in the smoothing capacitor 13 is discharged via the resistor 14. With the electric discharge, the electric potential level of the node N1 is shifted from the H level to the L level, as shown in FIG. 7.

That is, at the node N1 on the output end side of the magnetic field detecting section 6 of the present embodiment, the electric potential level is shifted from the L level to the H level almost immediately after the magnetic field was generated from the magnetic field generating section 7. The electric potential level is shifted from the H level to the L level, when the level of the magnetic field is attenuated to reach a level equal to or lower than a predetermined level.

Furthermore, an output signal having the electric potential level of the node N1 is inputted to the input end of the frequency divider circuit 15. In response to the signal, the electric potential level of the node N2 on the output end side of the frequency divider circuit 15 is shifted from the H level to the L level at the time t11a.

Following the shift of the electric potential level of the node N2 from the H level to the L level, the P-channel FET 9 is shifted from an off-state to an on-state, and thereby the power source section 8 starts supplying the driving power to each of the illuminating section 2, the image pickup section 3, and the wireless transmission section 4. That is, as shown in FIG. 7, the power source of the capsule endoscope 1 is turned on at the time t11 a.

As shown in FIG. 7, the electric potential level of the node N2 is maintained at the L level, even when the electric potential level of the node N1 was shifted from the H level to the L level. Accordingly, the electric potential level of the node N2 remains at the L level until the time t3a as the timing when the electric potential level of the node N1 is shifted again from the L level to the H level and also as the timing almost immediately after the time t13 when the magnetic field is generated again from the magnetic field generating section 7. As a result, the capsule endoscope 1 maintains the on-state during the period from the time t11 a to the time t13 a, as shown in FIG. 7.

Furthermore, as shown in FIG. 7, the electric potential level of the node N2 is shifted from the L level to the H level at the time t13a. Following the level shift, the P-channel FET 9 is shifted from the on-state to the off-state, and thereby the power source section 8 stops supplying the driving power to each of the illuminating section 2, the image pickup section 3, and the wireless transmission section 4. That is, as shown in FIG. 7, the power source of the capsule endoscope 1 is turned off at the time t13a.

That is, the capsule endoscope 1 of the present embodiment has a configuration and working such that on/off switching of the power source is performed every time the generation state of the magnetic field is switched from off to on in the magnetic field generating section 7.

Accordingly, the living body observation system 101 of the present embodiment enables easy of/off switching of the power source of the capsule endoscope 1 at a user's desired timing, thereby enabling easier control of exhaustion of the power source section 8 of the capsule endoscope 1 compared with a conventional system.

Furthermore, as described above, the living body observation system 101 of the present embodiment can reduce the power consumed in the magnetic field generating section 7 and enables a simple configuration of the magnetic field generating section 7.

Note that the on/off switching of the power source of the capsule endoscope 1 can be similarly performed not only in the case where the capsule endoscope 1 is disposed in the living body but also in the case where the capsule endoscope 1 is disposed outside the living body.

In addition, the respective embodiments described above are not limited to one applied to the capsule endoscope, but may be one applied to various living body information acquiring apparatuses having a configuration for acquiring living body information such as a temperature, pH or the like inside a living body.

Furthermore, in the above-described embodiments, a limiter circuit for suppressing the rise in the electric potential of the node N1 may be added.

It is needless to say that the present invention is not limited to the above-mentioned embodiments, and various changes and applications can be made therein without departing from the scope of the invention.

Cross-Reference to Related Application

The present invention is based on the priority of Japanese Patent Application No. 2008-111609 filed in Japan on April 22, 2008. The disclosure is referred to the description, claims, and drawings of the present invention.

**Claims**

**1.** A living body observation system comprising,
a living body information acquiring apparatus including:

a living body information acquiring section for acquiring living body information in a living body;
a wireless transmission section for wirelessly transmitting the living body information to outside of the living body;
a power source section for supplying driving power for driving the living body information acquiring section and the wireless transmission section;
a magnetic field detecting section for detecting a magnetic field from outside and outputting a detection result as an electric signal; and
a power supply control section for controlling a supplying state of the driving power supplied from the power source section to the living body information acquiring section and the wireless transmission section based on the electric signal; and

a magnetic field generating section disposed outside the living body information acquiring apparatus, the magnetic field generating section including:

a resonant circuit for generating a magnetic field by resonance; and
a driving circuit for supplying driving voltage for driving the resonant circuit.

2. The living body observation system according to Claim 1, wherein the driving circuit supplies voltage of rectangular waveform having a predetermined frequency to the resonant circuit as the driving voltage.

3. The living body observation system according to Claim 2, wherein a resonant frequency of the resonant circuit agrees with the predetermined frequency.

4. The living body observation system according to Claim 2, wherein the magnetic field detecting section includes a circuit capable of detecting the magnetic field generated from the magnetic field generating section at a resonant frequency that agrees with the predetermined frequency.

5. The living body observation system according to Claim 1, wherein the driving circuit supplies step-like voltage to the resonant circuit as the driving voltage.

6. The living body observation system according to Claim 1, wherein the living body information acquiring apparatus is a capsule endoscope.

7. A method of driving the living body observation system according to Claim 1, comprising switching a power source state of the living body information acquiring apparatus between on and off, every time the magnetic field is generated from the magnetic field generating section.

8. The method according to Claim 7, wherein the living body information acquiring apparatus is a capsule endoscope.

# FIG.1

# FIG.2

# FIG.3

# FIG.4

# FIG.5

# FIG.6

# FIG.7

## INTERNATIONAL SEARCH REPORT

| International application No. |
| --- |
| PCT/JP2009/055652 |

A. CLASSIFICATION OF SUBJECT MATTER
*A61B1/00(2006.01)i, A61B5/07(2006.01)i*

According to International Patent Classification (IPC) or to both national classification and IPC

B. FIELDS SEARCHED

Minimum documentation searched (classification system followed by classification symbols)
A61B1/00, A61B5/07

Documentation searched other than minimum documentation to the extent that such documents are included in the fields searched
Jitsuyo Shinan Koho     1922-1996   Jitsuyo Shinan Toroku Koho  1996-2009
Kokai Jitsuyo Shinan Koho  1971-2009   Toroku Jitsuyo Shinan Koho  1994-2009

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| X | WO 2007/083708 A1 (Olympus Medical Systems Corp.), 26 July, 2007 (26.07.07), Par. Nos. [0004], [0052] to [0070], [0096]; Claim 1; Figs. 1, 10 to 12 & US 2007/0191671 A1    & EP 1982635 A1 & KR 10-2008-0088635 A | 1-8 |
| X | JP 2005-287150 A (Olympus Corp.), 13 October, 2005 (13.10.05), Par. Nos. [0016] to [0033]; Figs. 2 to 4 & US 2007/0032697 A1    & WO 2005/093927 A & WO 2005/093927 A1    & CN 1938921 A | 1-8 |

| ☒ | Further documents are listed in the continuation of Box C. | ☐ | See patent family annex. |
| --- | --- | --- | --- |

| * | Special categories of cited documents: | | |
| --- | --- | --- | --- |
| "A" | document defining the general state of the art which is not considered to be of particular relevance | "T" | later document published after the international filing date or priority date and not in conflict with the application but cited to understand the principle or theory underlying the invention |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search 21 April, 2009 (21.04.09) | Date of mailing of the international search report 12 May, 2009 (12.05.09) |
| --- | --- |
| Name and mailing address of the ISA/ Japanese Patent Office | Authorized officer |
| Facsimile No. | Telephone No. |

Form PCT/ISA/210 (second sheet) (April 2007)

**INTERNATIONAL SEARCH REPORT**

| International application No. |
|---|
| PCT/JP2009/055652 |

C (Continuation). DOCUMENTS CONSIDERED TO BE RELEVANT

| Category* | Citation of document, with indication, where appropriate, of the relevant passages | Relevant to claim No. |
|---|---|---|
| X | JP 2005-81005 A (Olympus Corp.), 31 March, 2005 (31.03.05), Par. Nos. [0024] to [0039], [0052] to [0060]; Figs. 10 to 14 (Family: none) | 1-8 |
| X | JP 2005-237460 A (Olympus Corp.), 08 September, 2005 (08.09.05), Claims 1, 2, 5; Par. Nos. [0020] to [0028], [0035], [0054]; Figs. 2 to 6 (Family: none) | 1-8 |
| A | WO 2006/022365 A1 (Hokushin Denki Co., Ltd.), 02 March, 2006 (02.03.06), Claims 1, 2, 5, 6; Par. Nos. [0001], [0016] to [0025]; Fig. 1 & US 2007/0252441 A1 | 1-4 |
| A | JP 2004-159456 A (Kabushiki Kaisha Aiden Bideotoronikusu), 03 June, 2004 (03.06.04), Par. Nos. [0034] to [0067] (Family: none) | 1 |
| A | WO 2007/043458 A1 (Olympus Corp.), 19 April, 2007 (19.04.07), Par. Nos. [0051], [0057] to [0062]; Fig. 1 & EP 1932463 A1 & KR 10-2008-0043866 A & CN 101277640 A | 4 |
| A | JP 2008-79913 A (Olympus Medical Systems Corp.), 10 April, 2008 (10.04.08), Par. No. [0036]; Figs. 1 to 2 & WO 2008/038753 A1 | 4 |
| A | JP 2005-312236 A (Kanazawa University Technology Licensing Organization Ltd., Komatsu Power Tron Co., Ltd.), 04 November, 2005 (04.11.05), Par. Nos. [0009], [0016]; Fig. 2 (Family: none) | 5 |
| A | WO 2005/102452 A1 (Kanazawa University Technology Licensing Organization Ltd., Komatsu Power Tron Co., Ltd.), 03 November, 2005 (03.11.05), Par. Nos. [0019], [0023]; Fig. 3 & US 2007/0179576 A1 | 5 |

Form PCT/ISA/210 (continuation of second sheet) (April 2007)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2001224553 A

- JP 2008111609 A